# EUROPEAN PATENT SPECIFICATION

(11) **EP 2 046 947 B1**
(45) Date of publication and mention of the grant of the patent: **15.12.2010**
(21) Application number: 07789590.2
(22) Date of filing: 26.07.2007
(51) Int. Cl.: C12N 5/077

(54) **METHOD FOR THE PRODUCTION OF MULTIPOTENT STEM CELLS**
VERFAHREN ZUR HERSTELLUNG MULTIPOTENTER STAMMZELLEN
PROCEDE DE PRODUCTION DE CELLULES SOUCHES MULTIPOTENTES

(30) Priority: 28.07.2006 IT MI20061498
(43) Date of publication of application: 15.04.2009
(73) Proprietor: Metapontum Agrobios S.r.l., 75010 Metaponto (Matera) (IT)
(72) Inventor: CIFARELLI, Rosa, Anna, I-75010 Metaponto (Matera) (IT); CELLINI, Francesco, I-75010 Metaponto (Matera) (IT); DI LIDDO, Rosa, I-35131 Padova (IT); PARNIGOTTO, Pier, Paolo, I-35131 Padova (IT)
(74) Representative: De Gregori, Antonella
(86) International application number: PCT/IB2007/002204
(87) International publication number: WO 2008/012675

(56) References cited:
- TSUJI-TAKAYAMA KAZUE ET AL: "Demethylating agent, 5-azacytidine, reverses differentiation of embryonic stem cells." BIOCHEMICAL AND BIOPHYSICAL RESEARCH COMMUNICATIONS 8 OCT 2004, vol. 323, no. 1, 8 October 2004 (2004-10-08), pages 86-90, XP004537649 ISSN: 0006-291X cited in the application
- HATTORI NAOKO ET AL: "Epigenetic control of mouse Oct-4 gene expression in embryonic stem cells and trophoblast stem cells." THE JOURNAL OF BIOLOGICAL CHEMISTRY 23 APR 2004, vol. 279, no. 17, 23 April 2004 (2004-04-23), pages 17063-17069, XP002466544 ISSN: 0021-9258
- TAYLOR S M: "5-Aza-2'-deoxycytidine: cell differentiation and DNA methylation." LEUKEMIA : OFFICIAL JOURNAL OF THE LEUKEMIA SOCIETY OF AMERICA, LEUKEMIA RESEARCH FUND, U.K MAY 1993, vol. 7 Suppl 1, May 1993 (1993-05), pages 3-8, XP002066107 ISSN: 0887-6924
- LIN T-M ET AL: "ACCELERATED GROWTH AND PROLONGED LIFESPAN OF ADIPOSE TISSUE-DERIVED HUMAN MESENCHYMAL STEM CELLS IN A MEDIUM USING REDUCED CALCIUM AND ANTIOXIDANTS" STEM CELLS AND DEVELOPMENT, ELSEVIER, NL LNKD- DOI:10.1089/SCD.2005.14.92, vol. 14, no. 1, 1 January 2005 (2005-01-01), pages 92-102, XP009046469 ISSN: 1547-3287

## Description

The present invention relates to a new method for the production of multipotent stem cells.

Research on multipotent and totipotent stem cells (Conrad C., et al., 2005) has opened up new and interesting applicative prospects for cellular therapy and tissue engineering. What the most suitable stem cell source is for applicative purposes, however, is still to be defined.

The use of embryonic stem populations (ES) has recently stimulated a growing scientific interest in the medical field. Although these cells are characterized by a high replication capacity, differentiative pluripotentiality and long-term expansion *in vitro,* their therapeutic use, however, is limited due to the numerous ethical problems involved (Kiatpongsan S. et al., 2006).

Considerable experimental evidence has demonstrated that alternative sources of stem cells can be identified in adults; although these only have the capacity of differentiating in a limited number of cellular types, they do not arouse ethical problems. Among the sources of adult stem cells, bone marrow (Saulnier N. et al., 2005), seminiferous epithelium of the male gonad (Cyranoski D. et al., 2006), epithelia (Janes SM, et al., 2002), peripheral blood, cord blood (Saulnier N. et al., 2005) and adipose tissue (Strem BM, et al., 2005).

Adult stem cells, however, are not easily available as they are numerically limited; furthermore they cannot be cultivated *in vitro* for a long period as, after various cell divisions, they tend to lose their multipotentiality.

Together with this physiological stem cell sources, another extremely promising source has recently been added, which can be obtained by modification of the differentiated cell genetic program (F. Santos, et al., 2002; A.J. Peter, et al., 2001; R. Wolf, et al., 2001; J.C. Gutierrez, et al., 2000; T.H. Bestor, 2000; C. Stewart, et al., 1982; R.D. Palmiter, et al., 1982; D. Biniszkiewicz, et al., 2002; M.F. Chan, et al., 2001; M. Okano, et al., 1999). The researchers Wilmut and Campbell (Shiels PG, et al., 1999; Eyestone WH, et al., 1999) and subsequently Yanagimachi and collaborators (Wakayama T, et al., 2001) clearly established, using animal models, that the nucleus of completely differentiated somatic cells can be reprogrammed if transferred into the cytoplasm of an egg cell. The mechanisms and molecules involved in this deprogramming and reprogramming process of the genome of somatic cells has not yet been completely clarified. It is clear, however, that during this process the chromatin is decondensed, the nucleosome structure is destabilized and the regulator proteins are dissociated from the DNA fibre thus influencing the gene expression (T. Haaf, et al., 2000).

Other experimental observations have shown that, in the first embryonic development phases, there are numerous epigenetic methylation modifications of the cytosine residues with respect to the genome, as a result of which there is a change in the cell phenotype and commitment of the cell towards a specific differentiative line (F. Santos, et al., 2002; A.J. Peter, et al., 2001; R. Wolf, et al., 2001; J.C. Gutierrez, et al., 2000; Jetsche A. et al., 2006; Szyf M, et al., 1989; Keshhet I, et al., 1985).

In this respect, the idea was developed of being able to reprogram any completely differentiated cell by partially or completely modifying the methylation state of its genome, by means of treatment with specific factors.

Taranger and collaborators (Taranger CK, et al., 2005) have in fact shown that epithelial cells 293T can de-differentiate until markers are expressed typical of the embryonic state after 1 hour treatment with the extract of NCCIT cells, isolated from teratoma. Quantitative gene expression analysis and microarray study effected on the colonies maintained in a culture for 23 passages revealed that the transition to the pluripotent cell phenotype involves a dynamic stimulation of hundreds of genes typical of NCCIT cells (among which OCT4 (Ovitt CE, et al., 1998), SOX2 (M.V. Zappone, et al., 2000), NANOG (K. Mitsui, et al., 2003)) and an expression drop in genes characterizing cells 293T and generic differentiation indicators such as, for example, type A laminins.

Furthermore, experimental evidence has shown that a demethylating agent, 5' Aza 2' cytidine (5-AzaC), a product analogous to cytosine, can cause an extensive demethylation of the residues of 5-methylcytosine (M. Vlahovic, et al., 1999) and reduce the methyltransferase DNA activity (Tsuji-Takayama K, et al., 2004; Karpf AR, et al., 1999; U. Aapola, et al., 2001; X. Cheng, 1995; L.R. Silverman, et al., 1993; Simonsson, S., and Gurdon, 2004; Flasza, M., et al., 2003).

4 enzymes of DNA cytosine methyltransferase, Dnmt1, Dnmt3a, Dnmt3b and Dnmt31 have been identified in embryonic cells (Shiels PG, et al., 1999; Eyestone WH, et al., 1999; Wakayama T, et al., 2001).

Dnmt1 is the main enzyme which maintains the methylation state during the DNA replication. Its inactivation in murine models resulted in the loss of genomic imprinting and led to the precocious mortality of the embryo. Dnmt3a, Dnmt3b and Dnmt31, which mainly catalyze the methylation *de novo,* are extremely active in embryonic stem cells and promote their differentiation *in vitro.*

Used as an effective chemotherapeutic agent in the treatment of leukemia (Yang AS, et al., 2006), 5-AzaC has proved to be a useful experimental means for studying DNA methylation in cell differentiation and in gene activation mechanisms (J.G. Herman, et al., 1994; K. Yoshiura, et al., 1995; Y.L. Ottaviano, et al., 1994; C.M. Bender, et al., 1998). 5-AzaC, for example, has proved to intervene in differentiation induction in a myogenic sense of multipotent stem cells isolated from the umbilical cord, bone marrow and adipose tissue (Lin Y, et al., 2006). Other studies also provide data relating to its modulating activity on embryonic development with respect to proliferogenic and differentiative effects (Tsuji-Takayama K, et al., 2004). Furthermore, embryoid bodies differentiated *in vitro* and treated for 6 hours with 5-AzaC (1 µM) re-established the colony growth process typical of stem cells and the sensitivity to LEUKEMIA INHIBITORY FACTOR (LIF).

The demethylating action of 5-Azacitydine, with a consequent restoration of the cell multipotentiality, is widely demonstrated and verified also in plants, mono-cotyledons (wheat) and dicotyledons (tomatoes), in W02005003344.

Lin Tsai Ming et al. (Stem cells and development, vol. 14, no.1, February 2005, pages 92-102) disclose a method to obtain multipotential stem cells from adipose tissue using a medium with reduced calcium and antioxidants. The cells thus obtained are able to differentiate into adipocytes, osteoblasts, chondrocytes and myoblasts and express embryonic stem cell marker Oct-4. The cited document does not disclose a method wherein the multipotential stem cells are obtained after treatment with 5-azaC.

Morphological/functional analysis and gene expression study have lead to the conclusion that 5-AzaC reverses the differentiated state of embryonic cells, organized in embroid bodies, to that which can be phenotypically attributed to undifferentiated pluripotent stem cells expressing typical markers such as SSEA-1, alkaline phosphatase, Oct4, Nanog and SOX2.

Numerous other studies have described reactivation in somatic cells by treatment with 5-AzaC of silenced genes such as VHL, E-cadherin, estrogen receptor and p16. Genes of the STAT family, which have an important role in maintaining the undifferentiated state of embryonic stem cells (ES) and their capacity of self-replication by transduction of the LIF signal, are also probably the target of demethylating agents. Karpf and collaborators (Karpf AR., et al., 1999) have observed that silenced STAT genes in tumor cells of the colon are reactivated by treatment with 5-AzaC, accompanied by the reestablishment of the cell sensitivity with alpha interferon (INF-α).

It would therefore seem that treatment with 5'Aza 2' cytidine can have an effect on the proliferation, differentiation or de-differentiation of different cell types, belonging to animal or vegetable organisms and at various development phases (embryonic, adult, etc.).

In the light of what is specified above, there is evidently the necessity of availing of a method for the production of multipotent stem cells starting from an inexhaustible autologous cell source, easily available and free of ethical problems. It is also desirable that the method be easily controllable, repeatable and above all, that it does not lead to the neoplastic transformation of the same cells.

The authors of the present invention have now found a method for the production of multipotent stem cells which satisfies all the demands described above and which overcomes the disadvantages of the methods already known in the state of the art. The authors have in fact found advantageous alternative sources of highly differentiated somatic cells destined for generative medicine, which, subjected to demethylating treatment at suitable concentrations, represent an inexhaustible source of easily available multipotent autologous stem cells. The reprogramming of this somatic cell source represents an important solution to the problem of the scarcity of bone marrow donors and has no problems of an ethical nature.

An object of the present invention therefore relates to a method for the production of multipotent stem cells starting from adipocytes or pre-adipocytes comprising the demethylating treatment phase of said cells with 5' Aza 2' cytidine at a concentration ranging from 0.1 µM to 175 µM until the appearance of markers typical of the undifferentiated and multipotent embryonic state and/or the reduction in the expression of the late differentiative phase markers.

According to a preferred embodiment of the method according to the invention, when said method is carried out starting from pre-adipocytes it comprises the following phases:
a) induction of the differentiation of pre-adipocytes in mature cells by:
   i) culture of the pre-adipocytes until the maximum confluence is reached;
   ii) treatment with differentiating factors;
   iii) culture in maintenance medium free of differentiative induction agents until the expression of leptin or GLUT-4 is verified;
b) demethylating treatment of the highly differentiated mature adipose cells with 5' Aza 2' cytidine at a concentration ranging from 0.1 µM to 175 µM until the appearance of markers typical of the undifferentiated embryonic state and/or the reduction in the expression of the late differentiative phase markers.

In a particularly preferred embodiment of the invention, said highly differentiated adult somatic cells of mammals or their precursors are adipocytes or pre-adipocytes and said late differentiative phase markers are adipogenic markers, preferably selected from leptin and GLUT-4 or a combination thereof.

According to a preferred embodiment of the method according to the invention, when said method is carried out starting from pre-adipocytes, it comprises the following phases:
a) induction of the differentiation of said preadipocytes in mature adipocytes by:
   i) culture of the adipocyte precursors until the maximum confluence is reached;
   ii) treatment with differentiating factors, preferably selected from insulin, isobutylmethylxanthine, dexamethasone, indomethacin or a combination thereof ;
   iii) culture in a maintenance medium free of adipogenic induction agents until the expression of leptin or GLUT-4 is verified;
b) demethylating treatment of the highly differentiated mature adipose cells with 5' Aza 2' cytidine at a concentration ranging from 0.1 µM to 175 µM until the appearance of markers typical of the undifferentiated embryonic state and/or the reduction in the expression of the late differentiative phase adipogenic markers, preferably selected from leptin and GLUT-4 or a combination thereof. Said markers typical of the undifferentiated embryonic state are preferably selected from OCT4, SOX2, NANOG, SSEA-1 and alkaline phosphates or a combination thereof.

The concentration of use of 5' Aza 2' cytidine is preferably selected from 100 µM, 125 µM, 150 µM and 175 µM.

In a preferred embodiment, said culture in a maintenance medium free of adipogenic induction agents of phase iii) has a duration of 7-10 days.

According to a preferred embodiment, the method of the invention also comprises a differentiation induction phase in an osteogenic sense (100 nM dexamethasone, 10 mM β-glycerophosphate, 0.05 mM ascorbic acid-2-phosphate), chondrogenic (2.0x10⁻⁴ M ascorbic acid-2-phosphate, 1 ng/ml TGFβ1) or myogenic (20% Fetal Bovine Serum (FBS), 10% Horse Serum, 1% chicken embryo extract).

Finally, the invention relates to the use of 5' Aza 2' cytidine at a concentration ranging from 0.1 µM to 175 µM for the induction of multipotent staminality starting from adipocytes or pre-adipocytes. The concentration of 5' Aza 2' cytidine is preferably selected from 100 µM, 125 µM, 150 µM and 175 µM.

The present invention is now described for illustrative but non-limiting purposes, according to its preferred embodiments, with particular reference to the figures of the enclosed drawings, in which:
figure 1 shows the result of a semiquantitative PCR relating to the expression of the Ob (leptin) gene of populations of adipocytes treated with 175 µM 5-Azacytidine;

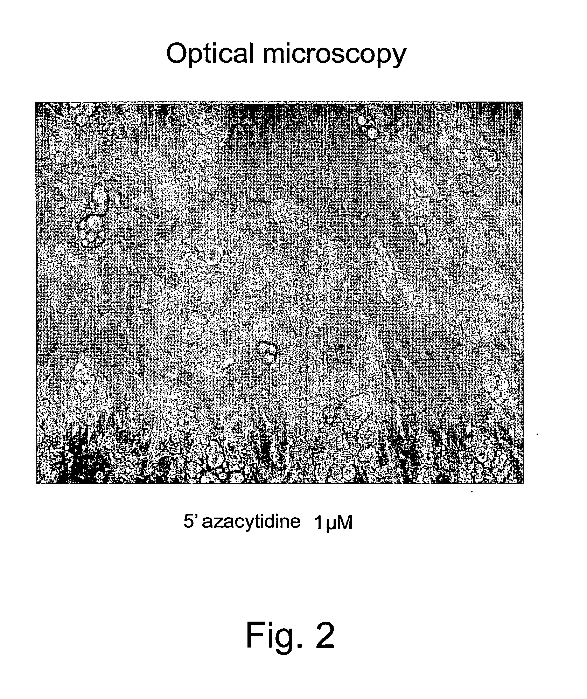

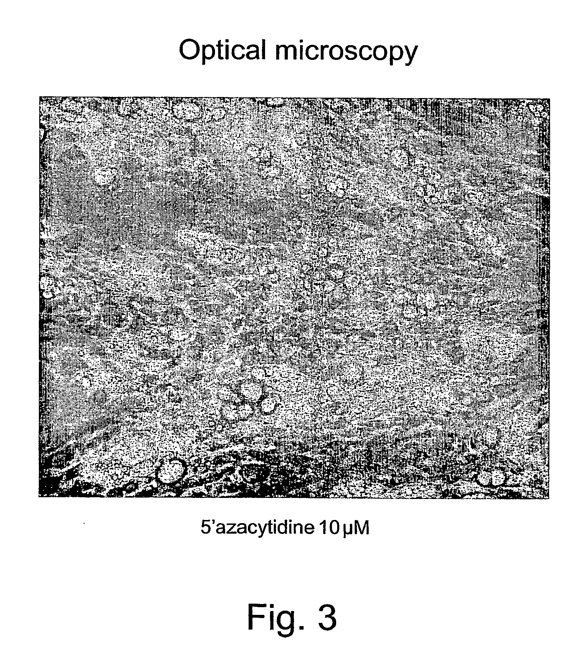

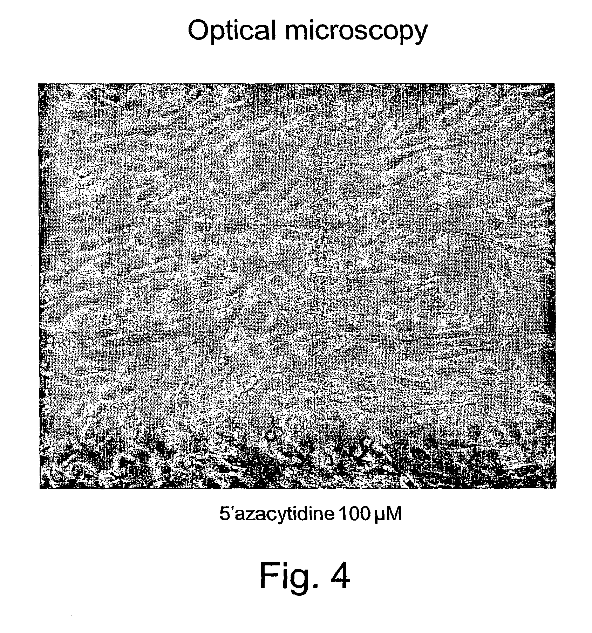

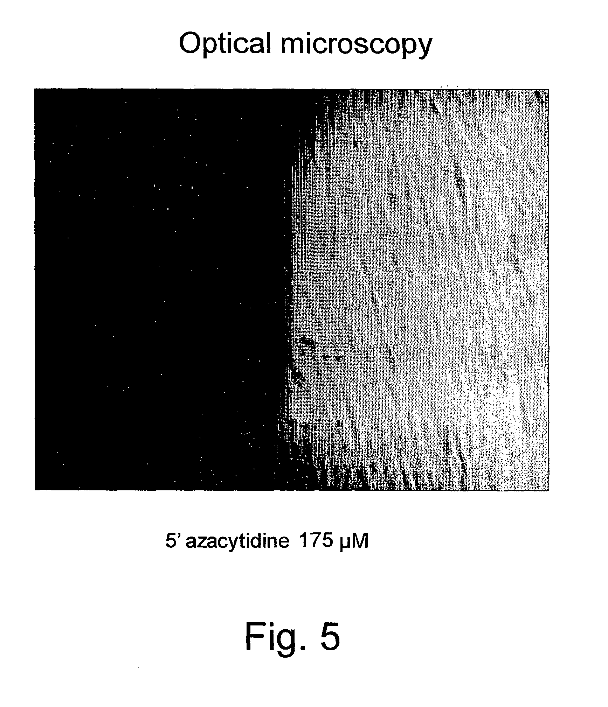

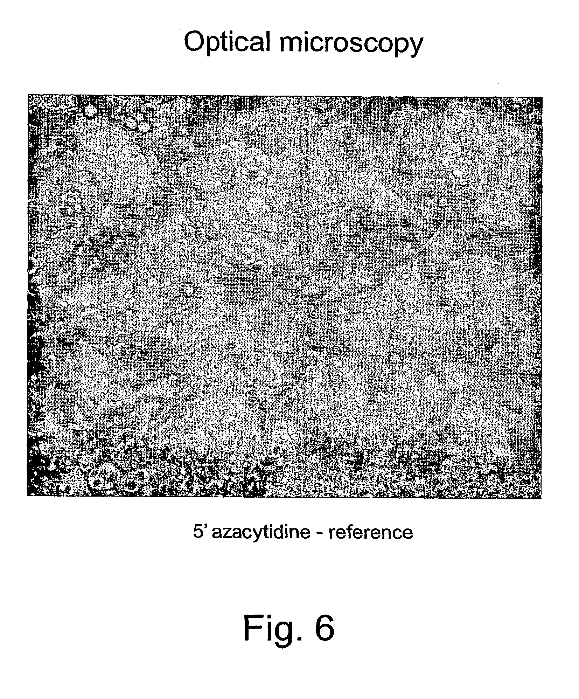

figures 2-6 show the phase contrast microscope images (100x enlargement) of the cell populations after 10 days of treatment with 5-Azacytidine (1 µM, 10 µM, 100 µM, 175 µM) and mature reference adipocytes, respectively;
figures 7 and 8 show the phase contrast microscope images (100x enlargement) of cultures treated with 5-Azacytidine at concentrations equal to 250 µM and lower than 0.1 µM, respectively.

### EXAMPLE 1

The effects were evaluated of the demethylating agent 5-Azacytidine (at different concentrations) on the capacity of mature adipose cells of reverting to the undifferentiated state and being reprogrammed, after adequate stimulation, in a multilinear differentiative sense (for example, in an osteogenic, chondrogenic and myogenic sense).

### MATERIALS AND METHODS

### Development of an adequate cell model in vitro

Human pre-adipocytes and embryonic cell lines 3T3-L1 were sown at a density of 5 x 10³ cells/cm² and cultivated in Petri plates in a proliferative medium (DMEM-F12, 10% Fetal Calf Serum (FCS), 1% antibiotic-fungizone (AF)) until the maximum cell confluence is reached. The differentiative induction is obtained by 48 hour treatment in specific medium consisting of DMEM, 10% Fetal Bovine Serum (FBS), 1% AF and specific differentiating factors (insulin, isobutylmethylxanthine, dexamethasone, indomethacin). The mature differentiated form (univacuolar phenotype) is reached after culture of 7-10 days in maintenance medium, free of adipogenic inductive agents. Immunofluorescence studies verify the expression of leptin, a hormone typically expressed by adipocytes in adult phase.

### 5-AzaC treatment

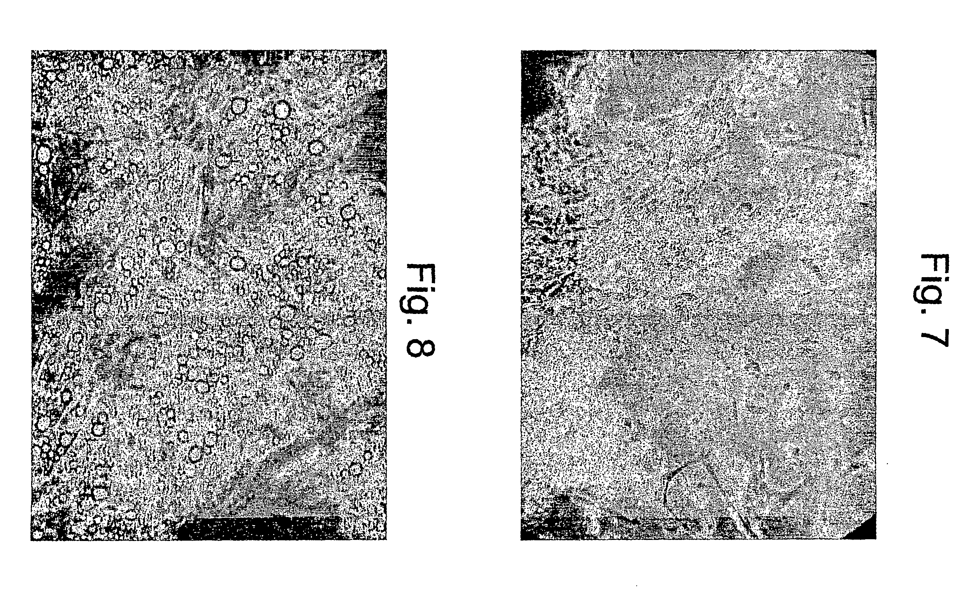

The mature adipose cells were maintained in a culture with DMEM, 10% FBS, 1% AF for two weeks, under continuous stimulation of 5'Azacytidine at molar concentrations of 0.1 µM, 1 µM, 10 µM, 100 µM, 125 µM, 150 µM, 175 µM, 250 µM. The optimum dose of 5'Azacytidine ranges from 0.1 µM to 175 µM and the concentrations which are particularly effective are those between 100 µM and 175 µM. At doses ≥ 250 µM the demethylating treatment is no longer suitable for the production of multipotent cells starting from highly differentiated cells as it becomes a chemotherapeutic treatment which induces the cells to apoptosis rather than reverting to the undifferentiated state as shown in Figure 7 where a high apoptotic degree is observed (treatment of cultures with 5'Azacytidine at a concentration of 250 µM). At doses ≤ 0.1 µM as shown in Figure 8, there are no signs of cell damage and/or cell proliferation in the cells treated.

### Expression of leptin: PCR reaction experimental conditions

In order to amplify the cDNA obtained from the 5-Aza cultures treated, the following were used:
- Eppendorf thermocycler Amplifier ;
- Specific primers designed with the help of the *PrimerQuest* program (http://biotools.idt.com) *and Nucleotide database* consultation (http://www.ncbi.nlm.nih.gov) and *BLAST* (Basic Local Alignment Search Tool) (http://www.ncbi.nlm.nih.gov/BLAST/).

The primers were purchased from New England Biolabs.

Amplification conditions: 95°C for 10 minutes (initial denaturation), 30 cycles of 95°C for 30 seconds (denaturation), 52°C for 45 seconds (annealing), 72°C for 45 seconds (extension), 72°C for 10 minutes (final extension). The reaction mixture was prepared with 1 µl of cDNA, 1 µl of dNtPs Mix (10 mM), 1.25 µl of MgCl₂ solution (25 mM), 1.25 µl of buffer 10X, 0.2 µl of Ampli Taq Gold (5U µl) (Applied Biosystem), 1 µl of specific sense primer and 1 µl of antisense primer (10 pM), 8 µl of H₂O RNase free in a final volume of 12.5 µl.

### RESULTS

### Morphological analysis of the cultures treated and control by means of optical microscopy

In cells treated with the demethylating agent 5-Azac, the preliminary results show a change in the morphology of the plate; it is mainly observed in fact at concentrations of 1-10 µM and higher than 100 µM, which, with the progressive disappearance of globose cells, grouped in large clusters and characterized by a voluminous lipid vacuole, is associated with the appearance of cell elements having a more elongated form with or without lipid vacuoles distributed at a cytoplasmatic level. Optic microscope images are provided hereunder of cell populations after 10 days of treatment with 5-AzaC. Phase contrast microscope images (100x enlargement) are enclosed of reference populations and those treated with 5-Azacytidine.

### Cell growth determination

Cell proliferation is observed by measuring the 5'-bromo-2'deoxyuridine (BrdU) incorporated in the adipose cells treated with 5-Azacytidine. As it is known in literature that mature adipose cells do not have a proliferating capacity, it is interesting to better characterize the cell fraction having a fibroblastoid morphology which only appears in the samples treated and not in the reference samples.

### Determination of cell apoptosis

With the use of the TACS Klenow In Situ Apoptotic Detection kit, apoptotic phenomena were observed in both the samples treated and in the reference samples 24 hours, 48 hours, 72 hours after treatment with 5-Azacytidine. The following table indicates the results relating to the samples of adipocytes treated with 125 µM, 150 µM, 175 µM.

**Table 1**

| TIME | REFERENCE | TREATED | TREATED | TREATED |
|---|---|---|---|---|
| | | 125 µM | 150 µM | 175 µM |
| 25 hours | 10% | 15% | 22% | 38% |
| 48 hours | 12% | 15% | 28% | 41% |
| 72 hours | 10% | 10% | 10% | 10% |

| | | | | |
|---|---|---|---|---|
| *Semiquantitative (PCR) and quantitative (RT-PCR) tests* | | | | |

The expression of the late-phase adipogenic markers (leptin, GLUT-4), precocious-phase markers (PPAR-y, CEBP-α) and markers typical of the undifferentiated embryonic state (OCT4, SOX2, NANOG, SSEA-1, alkaline phosphatase) was evaluated by means of PCR and RT-PCR.

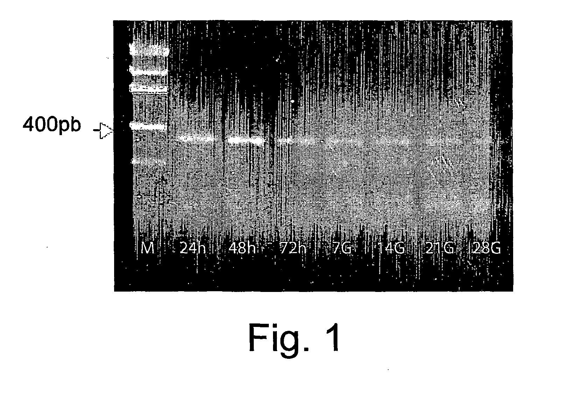

The preliminary results show a reduction in the expression of the differentiative late-phase adipogenic markers. Figure 1 shows the result of the semiquantitative PCR relating to the expression of the Ob gene (leptin) of adipocyte populations treated with 175 µM 5-Azacytidine.

### BIBLIOGRAPHY

- Conrad C, Huss R. J Surg Res. 2005 Apr; 124(2):201-8.
- Kiatpongsan S, Tannirandorn Y, Virutamasen P. J Med Assoc Thai. 2006 Jan;89(1):111-7.
- Saulnier N, Di Campli C, Zocco MA, Di Gioacchino G, Novi M, Gasbarrini A. Eur Rev Med Pharmacol Sci. 2005 Nov-Dec;9(6):315-24.
- Cyranoski D. Nature. 2006 Mar 30;440(7084):586-7.
- Janes SM, Lowell S, Hutter C. J Pathol. 2002 Jul; 197(4):479-91.
- Strem BM, Hicok KC, Zhu M, Wulur I, Alfonso Z, Schreiber RE, Fraser JK, Hedrick MH. Keio J Med. 2005 Sep; 54(3):132-41.
- F. Santos, B. Hendrich, W. Reik, W. Dean. Dev. Biol. 2002; 241:172-182.
- A.J. Peter, T. Daiya. Science 2001;293:1068-1070.
- R. Wolf, W. Dean, J. Walter. Science 2001; 293:1089-1093.
- J.C. Gutierrez, S. Callejas, S. Borniquel, A. Martin-Gonzalez. Int. Microbiol. 2000; 3:139-146.
- T.H. Bestor. Hum. Mol. Genet. 2000; 9:2395-2402.
- C. Stewart, H. Stuhlmann, D. Jahner, R. Jaenisch. Proc. Natl. Acad. Sci. USA 1982; 79:4098-4102.
- R.D. Palmiter, H.Y. Chen, R.L. Brinster. Cell 1982; 29:701-710.
- D. Biniszkiewicz, J. Gribnau, B. Ramsahoye, F. Gaudet, K. Eggan, D. Humpherys, M.A. Mastrangelo, Z. Jun, J. Walter, R. Jaenisch. Mol. Cell. Biol. 2002; 22:2124-2135.
- M.F. Chan, R. van Amerongen, T. Nijjar, E. Cuppen, P.A. Jones, P.W. Laird. Mol. Cell. Biol. 2001; 21:7587-7600.
- M. Okano, D.W. Bell, D.A. Haber, E. Li. Cell 1999; 99:247-257.
- Shiels PG, Kind AJ, Campbell KH. Wilmut I, Waddington D, Colman A, Schnieke AE. Cloning. 1999; 1(2):119-25.
- Eyestone WH, Campbell KH. J Reprod Fertil Suppl. 1999; 54:489-97.
- Wakayama T, Yanagimachi R. Mol Reprod Dev. 2001 Apr; 58(4):376-83.
- T. Haaf, M. Schmid. Cytogenet. Cell Genet. 2000; 91:113-123.
- F. Santos, B. Hendrich, W. Reik, W. Dean. Dev. Biol. 2002; 241:172-182.
- A.J. Peter, T. Daiya. Science 2001; 293:1068-1070.
- R. Wolf, W. Dean, J. Walter. Science 2001; 293:1089-1093.
- J.C. Gutierrez, S. Callejas, S. Borniquel, A. Martin-Gonzalez. Int. Microbiol. 2000; 3:139-146.
- Jetsch A. Curr Top Microbiol Immunol. 2006;301:203-25.
- Szyf M, Schimmer BP, Seidman JG. Proc Natl Acad Sci U S A. 1989 Sep; 86(18):6853-7.
- Keshhet I, Yisraeli J, Cedar H. Proc Natl Acad Sci U S A. 1985 May; 82(9):2560-4.
- Taranger CK, Noer A, Sorensen AL, Hakelien AM, Boquest AC, Collas P. Mol Biol Cell. 2005; 16(12):5719-35.
- Ovitt CE, Scholer HR. Mol Hum Reprod. 1998 Nov; 4(11):1021-31.
- M.V. Zappone, R. Galli, R. Catena, N. Meani, S. De Biasi, E. Mattei, C. Tiveron, A.L. Vescovi, R. Lovell-Badge, S. Ottolenghi. Development 2000; 127:2367-2382.
- K. Mitsui, Y. Tokuzawa, H. Itoh, K. Segawa, M. Murakami, K. Takahashi, M. Maruyama, M. Maeda, S. Yamanaka. Cell 2003; 113:631-642.
- M. Vlahovic, F. Bulic-Jakus, G. Juric-Lekic, A. Fusis, S. Maric, D. Serman. Int. J. Dev. Biol. 1999; 43:843-846.
- Tsuji-Takayama K, Inoue T, Ijiri Y, Otani T, Motoda R, Nakamura S, Orita K. Biochem Biophys Res Commun. 2004 Oct 8; 323(1):86-90.
- Karpf AR, Peterson PW, Rawlins JT, Dalley BK, Yang Q, Albertsen H, Jones DA. Proc Natl Acad Sci U S A. 1999 Nov;96(24):14007-12.
- U. Aapola, R. Lyle, K. Krohn, S.E. Antonarakis, P. Peterson. Cytogenet. Cell Genet. 2001;92:122-126.
- X. Cheng. Curr. Opin. Struct. Biol. 1995;5:4-10.
- L.R. Silverman, J.F. Holland, R.S. Weinberg, B.P. Alter, R.B. Davis, R.R. Ellison, E.P. Demakos, C.J. Cornell, R.W. Carey, C. Schiffer, E. Frei III, O.R. McIntypr. Leukemia (Baltimore) 1993; 7:21-29.
- Simonsson, S., and Gurdon. J. Nat. Cell Biol. 2004; 6:984-990.
- Flasza, M., Shering, A. F., Smith, K., Andrews, P. W., Talley, P., and Johnson, P. A. Cloning Stem Cells 2003; 5:339-354.
- Yang AS, Doshi KD, Choi SW, Mason JB, Mannari RK, Gharybian V, Luna R, Rashid A, Shen L, Estecio MR, Kantarjian HM, Garcia-Manero G, Issa JP. Cancer Res. 2006 May; 66(10):5495-503.
- J.G. Herman, F. Latif, Y. Weng, M.I. Lerman, B. Zbar, S. Liu, D. Samid, D.S. Duan, J.R. Gnarra, W.M. Linehan, S.B. Baylin. Proc. Natl. Acad. Sci. USA 1994; 919:700-9704.
- K. Yoshiura, Y. Kanai, A. Ochiari, Y. Shimoyama, T. Sugimura, S. Hirohashi. Proc. Natl. Acad. Sci. USA 1995; 92:7416-7419.
- Y.L. Ottaviano, J.P. Issa, F.F. Parl, H.S. Smith, S.B. Baylin, N.E. Davidson. Cancer Res. 1994; 54:2552-2555.
- C.M. Bender, M.M. Pao, P.A. Jones. Cancer Res. 1998; 58:95-101.
- Lin Y, Liu L, Li Z, Qiao J, Wu L, Tang W, Zheng X, Chen X, Yan Z, Tian W. Mol Cell Biochem. 2006 May 23.
- Tsuji-Takayama K, Inoue T, Ijiri Y, Otani T, Motoda R, Nakamura S, Orita K. Biochem Biophys Res Commun. 2004 Oct 8; 323(1):86-90.
- Cellini Francesco, Cifarelli RosaAnna Gallitelli Maria; Mango Teresa; Lauria Giuseppe; Semeraro Lucia. METHOD FOR THE ISOLATION OF EXPRESSED SEQUENCE TAGS IN PLANTS 13.01.2005. WO2005003344

## Claims

1. A method for the production of multipotent stem cells starting from adipocytes or pre-adipocytes comprising the demethylating treatment phase of said cells with 5' Aza 2' cytidine at a concentration ranging from 0.1 µM to 175 µM until the appearance of markers typical of the undifferentiated and multipotent embryonic state and/or the reduction in the expression of the late adipogenic differentiative phase markers.

2. The method according to claim 1, comprising the following phases:
a) induction of the differentiation of pre-adipocytes in mature adipocytes by:
i) culture of the pre-adipocytes until the maximum confluence is reached;
ii) treatment with differentiating factors;
iii) culture in a maintenance medium free of adipogenic induction agents until the expression of leptin or GLUT-4 is verified;
b) demethylating treatment of the highly differentiated mature adipose cells with 5' Aza 2' cytidine at a concentration ranging from 0.1 µM to 175 µM until the appearance of markers typical of the undifferentiated embryonic state and/or the reduction in the expression of the late differentiative phase adipogenic markers.

3. The method according to any of the claims 1-2, wherein the concentration of 5' Aza 2' cytidine is selected from 100 µM, 125 µM, 150 µM, 175 µM.

4. The method according to any of the claims 1-3, wherein said markers typical of the undifferentiated embryonic state are selected from OCT4, SOX2, NANOG, SSEA-1 and alkaline phosphatase or a combination thereof.

5. The method according to any of the claims 1-4, wherein said adipogenic late differentiative phase markers are selected from leptin and GLUT-4 or a combination thereof.

6. The method according to any of the claims 2-5, wherein said differentiating factors of phase ii) are selected from insulin, isobutylmethylxanthine, dexamethasone, indomethacin or a combination thereof.

7. The method according to any of the claims 2-6, wherein said culture of phase iii) has a duration of 7-10 days.

8. The method according to any of the claims 1-7, further comprising a differentiation induction phase in an osteogenic, chondrogenic or myogenic sense.

9. Use of 5' Aza 2' cytidine at a concentration ranging from 0.1 µM to 175 µM for the induction of multipotent staminality starting from adipocytes or pre-adipocytes

10. Use according to claim 9, wherein the concentration of 5' Aza 2' cytidine is selected from 100 µM, 125 µM, 150 µM, 175 µM.

## Patentansprüche

1. Ein Verfahren zur Herstellung von von Adipozyten oder Präadipozyten ausgehenden multipotenten Stammzellen, umfassend eine demethylierende Behandlungsphase dieser Zellen mit 5' Aza 2' Cytidin bei einer Konzentration, die von 0.1 µM bis 175 µM reicht, bis zur Erscheinung von Markierungen typisch für den undifferenzierten und multipotenten embryonalen Zustand und/oder bis zur Herabsetzung der Expression der späten fettbildenden Differenzierungsphasemarkierungen.

2. Verfahren nach Anspruch 1, die folgenden Phasen umfassend:
a) Induktion der Differenzierung der Präadipozyten in reifen Adipozyten durch:
i) Kultur der Präadipozyten bis die maximale Konfluenz erreicht ist;
ii) Behandlung mit Differenzierungsfaktoren;
iii) Kultur in einem Versorgungsmedium, das frei von Induktionsstoffen ist, bis die Expression von Leptin oder GLUT-4 nachgewiesen ist;
b) demethylierende Behandlung der hochdifferenzierten reifen Fettzellen mit 5' Aza 2' Cytidin bei einer Konzentration, die von 0.1 µM bis 175 µM reicht, bis zur Erscheinung von Markierungen typisch für den undifferenzierten embryonalen Zustand und/oder bis zur Herabsetzung der Expression der späten Differenzierungsphase fettbildenden Markierungen.

3. Verfahren nach irgendeinem der Ansprüche 1 bis 2, worin die Konzentration von 5' Aza 2' Cytidin aus 100 µM, 125 µM, 150 µM, 175 µM ausgewählt wird.

4. Verfahren nach irgendeinem der Ansprüche 1 bis 3, worin die Markierungen, die typisch für den undifferenzierten embryonalen Zustand sind, aus OCT4, SOX2, NANOG, SSEA-1 und alkalischen Phosphatasen, oder einer Kombination davon, ausgewählt werden.

5. Verfahren nach irgendeinem der Ansprüche 1 bis 4, worin die späten fettbildenden Differenzierungsphasemarkierungen aus Leptin und GLUT-4, oder einer Kombination davon, ausgewählt werden.

6. Verfahren nach irgendeinem der Ansprüche 2 bis 5, worin die Differenzierungsfaktoren der Phase ii) aus Insulin, Isobutylmethylxanthin, Dexamethason, Indomethacin, oder einer Kombination davon, ausgewählt werden.

7. Verfahren nach irgendeinem der Ansprüche 2 bis 6, worin die Kultur in Phase iii) eine Dauer von 7 bis 10 Tagen hat.

8. Verfahren nach irgendeinem der Ansprüche 1 bis 7, außerdem eine Differenzierungsinduktionsphase in einem osteogenen, chondrogenen oder myogenen Sinn umfassend.

9. Verwendung von 5' Aza 2' Cytidin bei einer Konzentration, die von 0.1 µM bis 175 µM reicht, für die Induktion von von Adipozyten oder Präadipozyten ausgehenden multipotenten Stammzellen.

10. Verwendung nach Anspruch 9, worin die Konzentration von 5' Aza 2' Cytidin aus 100 µM, 125 µM, 150 µM, 175 µM ausgewählt wird.

## Revendications

1. Procédé de production de cellules souches multipotentes à partir d'adipocytes ou préadipocytes comprenant l'étape de traitement de déméthylation desdites cellules avec 5' Aza 2' cytidine à une concentration allant de 0,1 µM à 175 µM jusqu'à l'apparition de marqueurs typiques de l'état embryonnaire indifférencié et multipotent et/ou la réduction de l'expression des marqueurs de phase de différenciation adipocytaire terminale.

2. Procédé selon la revendication 1, comprenant les étapes suivantes :
a) induction de la différenciation de préadipocytes en adipocytes murs par:
i) la culture des préadipocytes jusqu'à ce que la confluence maximale soit atteinte ;
ii) le traitement avec des facteurs de différenciation ;
iii) la culture dans un milieu de maintien dépourvu d'agents d'induction adipocytaire jusqu'à vérification de l'expression de leptine ou GLUT-4 ;
b) le traitement de déméthylation des adipocytes murs hautement différenciés avec 5' Aza 2' cytidine à une concentration allant de 0,1 µM à 175 µM jusqu'à l'apparition de marqueurs typiques de l'état embryonnaire indifférencié et/ou la réduction de l'expression des marqueurs de phase de différenciation adipocytaire terminale.

3. Procédé selon l'une quelconque des revendications 1-2, dans lequel la concentration de 5' Aza 2' cytidine est choisie parmi 100 µM, 125 µM, 150 µM, 175 µM.

4. Procédé selon l'une quelconque des revendications 1-3, dans lequel lesdits marqueurs typiques de l'état embryonnaire indifférencié sont choisis parmi OCT4 , SOX2, NANOG, SSEA-1 et phosphatase alcaline ou une combinaison de ceux-ci.

5. Procédé selon l'une quelconque des revendications 1-4, dans lequel lesdits marqueurs de phase de différenciation adipocytaire terminale sont choisis à partir de leptine et GLUT-4 ou une combinaison de ceux-ci.

6. Procédé selon l'une quelconque des revendications 2-5, dans lequel lesdits facteurs de différenciation de l'étape ii) sont choisi à partir d'insuline, iso-butylméthylxanthine, dexaméthasone, indométhacine ou une combinaison de celles-ci.

7. Procédé selon l'une quelconque des revendications 2-6, dans lequel ladite culture de l'étape iii) a une durée de 7-10 jours.

8. Procédé selon l'une quelconque des revendications 1-7, comprenant en outre une étape d'induction de différenciation dans un sens ostéogénique, chondrogénique ou myogénique.

9. Utilisation de 5' Aza 2' cytidine à une concentration allant de 0,1 µM à 175 µM pour l'induction de cellules souches multipotentes à partir d'adipocytes ou préadipocytes.

10. Utilisation selon la revendication 9, dans laquelle la concentration de 5' Aza 2' cytidine est choisie parmi 100 µM, 125 µM, 150 µM, 175 µM.
